Europäisches Patentamt

European Patent Office

Office européen des brevets

(11) Veröffentlichungsnummer: **0 403 748 B1**

## EUROPÄISCHE PATENTSCHRIFT

(45) Veröffentlichungstag der Patentschrift: **25.01.95**

(51) Int. Cl.6: **A61K 9/20**, A61K 9/28

(21) Anmeldenummer: **90106947.6**

(22) Anmeldetag: **11.04.90**

Die Akte enthält technische Angaben, die nach dem Eingang der Anmeldung eingereicht wurden und die nicht in dieser Patentschrift enthalten sind.

(54) **Orale Lipidarzneiform.**

(30) Priorität: **19.06.89 DE 3919982**

(43) Veröffentlichungstag der Anmeldung:
**27.12.90 Patentblatt 90/52**

(45) Bekanntmachung des Hinweises auf die Patenterteilung:
**25.01.95 Patentblatt 95/04**

(84) Benannte Vertragsstaaten:
**AT BE CH DE ES FR GB IT LI NL SE**

(56) Entgegenhaltungen:
EP-A- 0 086 468     EP-A- 0 107 085
EP-A- 0 225 189     EP-A- 0 302 370
BE-A- 861 612       DE-A- 2 631 214
FR-A- 2 365 338

(73) Patentinhaber: **pharmed Dr. Liedtke GmbH**
**Tölzer Strasse 37**
**D-82031 Grünwald (DE)**

(72) Erfinder: **Liedtke, Rainer K., Dr.**
**c/o pharmed GmbH**
**Postfach 306**
**D-8022 Grünwald (DE)**

(74) Vertreter: **Bühling, Gerhard, Dipl.-Chem. et al**
**Patentanwaltsbüro**
**Tiedtke-Bühling-Kinne & Partner**
**Bavariaring 4**
**D-80336 München (DE)**

**Beschreibung**

Die Erfindung betrifft eine orale Lipidarzneiform insbesondere zur Verbesserung der Resorption von Wirkstoffen, die nach oraler Applikation nicht ausreichend bioverfügbar sind.

Es ist bekannt, daß zahlreiche Arzneistoffe nach oraler Applikation mit den bisher verfügbaren oralen Arzneiformen nicht ausreichend zur Resorption gebracht werden können. Hierzu gehören u.a. verschiedene Antibiotika sowie insbesondere Arzneistoffe mit Peptid- oder Proteinstruktur. Letztere können bisher nur parenteral appliziert werden, da sie nach oraler Gabe bereits im Gastrointestinaltrakt, noch vor Erreichen des Resorptionsortes, biochemisch inaktiviert werden. Das bekannteste Beispiel hierzu ist das blutzuckersenkende Proteohormon Insulin.

Insbesondere von rektalen und dermalen Applikationen ist bekannt, daß Trägergrundlagen, die u.a. unterschiedliche Mischungen von Lipiden enthalten zu signifikanten Resorptionsunterschieden bei den enthaltenen Wirkstoffen führen können. Lipidmischungen dieser Art besitzen, in Abhängigkeit von Art und Anteil der eingesetzten Lipide, d.h. Glycerinestern kurz-, mittel- oder langkettiger gesättigter oder ungesättigter Fettsäuren, die ihrerseits auch noch als Monoacylglyceride sowie einsäurige oder gemischtsäurige Di,-und Triacylglyceride vorliegen können, ein entsprechend unterschiedliches Löslichkeitsverhalten als auch unterschiedliches Lösungsverhalten für Wirkstoffe. Quantitative und qualitative unterschiedliche Relationen der Lipidmischungen können demnach Wirkstoffresorptionen beträchtlich beeinflussen. Entsprechend den resultierenden Hydrophilie/Lipophilie- Verhältnissen, müssen die Wirkstoffe auch in spezifischer pharmazeutisch-technischer Form in die Lipidmassen eingearbeitet werden. Rektale und dermale Applikationen erfüllen aber, auf Grund ihrer spezifischen pharmakokinetischen Profile und anatomischer Besonderheiten, nicht in allen Fallen die Voraussetzungen rascher Bioverfügbarkeit wie dies bei einigen Arzneistoffen wünschenswert oder erforderlich ist. Versuche zur Anwendung ausschließlich auf Fettbasis bestehender oraler Formulierungen, inbesondere mit Triacylglyceriden als Tabletten, gepressten Fettgranulaten oder kleineren Fettkügelchen (Fettpellets) mit hohem Schmelzpunkt zur Erreichung retardierter Freisetzungen durch Diffusion oder enzymatische Erosion, sind schon des längeren bekannt. Diese Formulierungen erfordern jedoch eine spezifische Herstellungstechnologie, sind primär nur für lipophile Arzneistoffe geeignet und zeigen gegenüber üblichen oralen Arzneiformulierungen, z.B. hydrophilen Tabletten keine Vorteile in der Bioverfügbarkeit. Sie fanden daher auch keinen breiten therapeutischen Einsatz. Bekannt sind auch Versuche zum Einsatz sogenannter Liposomen, d.h. vorzugsweise unter Einsatz von Phospholipiden, u.a. Phosphatidylcholin, hergestellten kleinsten sphärischen Lipidpartikeln (Nanopartikel), die als Arzneistoffe einschließende Vehikel verwandt werden sollen. Die exakte Herstellung dieser Partikel ist technisch kompliziert, insbesondere zeigen aber derartige Liposomen beträchtliche Stabilitätsprobleme.

Als eine technische Variante hierzu sind auch Versuche in Richtung hochdisperser, primär Triacylglycerid- haltiger Partikel (Micropellets) zu betrachten, die z.B. auch in Puderform vorliegen.

Auch der Einsatz von Mono- bzw. Mono- und Diglyceride enthaltenden Arzneiformen wurde bereits beschrieben. So nennt DE-A-2 631 214 die Verwendung von bei Körpertemperatur flüssigen Monoglyceriden, BE-A-861 612 und FR-A-2 365 338 offenbaren Lösungen oder mikrokristalline Suspensionen, welche Mono- und/oder Diglyceride enthalten.

Ein weiteres Problem verschiedener Fettmischungen ist, daß sie instabile Modifikationen zeigen können und hierdurch entsprechend lagerungsempfindlich sind. Dies betrifft insbesondere Fettmischungen z.B. auf Basis der Kakaobutter. Der Einsatz ungesättigter Fettsäuren in Fettmischungen wie z.B. Ölsäure (C18:1), Linolsäure (C18:2) und Linolensäure (C18:3) kann zudem oxidativ bedingte Haltbarkeitsprobleme mit Gefahr von Ranziditätserscheinungen beinhalten. Längerkettige Triacylglyceride besitzen auch relativ ungünstigere Lösungs- und Löslichkeitseigenschaften und sind daher primär nur für stärker lipophile Arzneistoffe geeignet. Sie werden zudem im Gastrointestinum, anders als Glycerinester kürzerkettiger Fettsäuren, offensichtlich nach Resorption auch überwiegend über lymphatische Wege abtransportiert. Auf Grund der deutlich geringeren Fluxraten in den Lymphwegen bedeutet dies insgesamt auch eine langsamere Resorption. Zudem werden längerkettige Triacylglyceride von gastrointestinalen Lipasen stärker als mittelkettige hydrolisiert.

Der Erfindung liegt die Aufgabe zugrunde, die Resorption von Wirkstoffen, die nach oraler Applikation nicht ausreichend bioverfügbar sind zu verbessern.

Diese Aufgabe wird erfindungsgemäß dadurch gelöst, daß die orale Lipidarzneiform aus der Kombination einer halbfesten, lipophilen Komponente mit einer festen, wasserlöslichen Komponente gebildet wird, wobei die halbfeste Komponente eine als Hartfett vorliegende homogene Lipidmischung mit thermisch reversiblem Fest/Flüssig-Schmelzverhalten, die noch unterhalb der Körpertemperatur von 37 Grad Celsium zu mindestens 95 % in flüssigem Aggregatzustand vorliegt und aus Monoacyl-, Diacyl-und Triacylglyceriden gesättigter Pflanzenfettsäuren der Kettenlängen von 6 bis 18 C Atomen, vorzugsweise einer Mischung von 40 bis 60 % Monoacyl- und Diacylglyceriden mit 40 bis 60 % Triacylglyceriden, besteht, in der die Wirkstoffe entweder direkt gelöst, suspendiert oder emulgiert vorliegen, wobei die feste Komponente der Arzneiform aus einer die gesamte Lipidmasse einschließenden, nicht diffusiblen, wasserlöslichen und mit der Lipidmasse chemisch nicht verbundenen Hülle vorzugsweise aus Hart-, Weichgelatine oder Stärke besteht, die auch eine Schutzschicht gegen den Magensaft enthalten kann.

Die Lipidmischung liegt vorzugsweise bei der physiologischen menschlichen Körpertemperatur um 37 Grad Celsius zu mindestens 95 % in flüssigem Aggregatzustand vor.

Um die Relationen der Fettsäuren im Lipidgemisch weiter zu optimieren, betragen, in einer weiteren Ausbildung der Erfindung, die im Gesamtlipidgemisch eingesetzten prozentualen Anteile an Fettsäuren für Caprylsäure 10-18%, für Caprinsäure 5-15%, für Laurinsäure 45-55%, für Myristinsäure 9-15%, für Palmitinsäure 3-10% und für Stearinsäure 3-10%, und diese Fettsäuren als Bestandteile von sowohl Monoacyl-, Diacyl- oder Triacylglyceriden vorliegen.

Als eine Mischung für eine derartige Lipidformulierung, wobei diese nur als weitere Erläuterung der Erfindung dient ohne daß diese hierauf beschränkt ist, sei eine Zusammensetzung angeführt bei der die prozentualen Anteile der eingesetzten Fettsäuren betragen: 16% Caprylsäure, 13% Caprinsäure, 50% Laurinsäure, 11% Myristinsäure, 5% Palmitinsäure und 5% Stearinsäure. Die Bestimmung der Fettsäuren im Lipidgemisch erfolgt durch Gaschromatografie (GC) mittels der Fettsäuremethylester. Eine Acetylierung der Lipidmasse führt zu Glyceriden, die dann direkt der GC zugeführt werden können. Die Auswertung der Peaks des GC ergibt ein quantitatives Bild der enthaltenen Monoacyl-, Diaycl- und Triacylglyceride, wobei die Partialglyceride Acetylgruppen aufweisen. Der Nachweis des geeigneten Schmelzverhaltens dieser Lipidmasse lässt sich entweder durch Bestimmung des Steigschmelzpunktes, des Tropfpunktes oder mittels Thermoanalyse festellen. Die Thermoanalyse gibt hierbei den gesamten Schmelzverlauf bei Aufnahme von Wärme wieder. Mit der Thermonanalyse lässt sich für eine Lipidzusammensetzung vorgenannter Art nachweisen, daß bei ca. 24 °C (Celsius) erst noch ein sehr geringer Teil der Lipidmasse geschmolzen ist, daß der Hauptteil des Schmelzvorganges um 30 °C liegt und nur noch ein geringer Anteil um 37 °C schmilzt. Die vergleichsweise durchgeführte Messung des Steigschmelzpunktes allein zeigt demgegenüber hierzu einen Wert von ca. 36 °C. Ein geeigneter Solid Fat-Index (Solid Fat Content, SFC) für eine dieserart zusammengesetzte Lipidmasse lässt sich mittels Puls-Kernresonanz Messung (NMR) ermitteln. Gemessen werden hierbei die Partikel, die bei bestimmten Temperaturen noch fest sind. Hierzu werden die Lipide exakter Vorbehandlung unterzogen: 3 g einer Probe werden 30 Minuten bei 70 °C klar aufgeschmolzen, 60 Minuten bei 0 °C gelagert und 35 Minuten bei Meßtemperatur gelagert. Ein aus 3 Messungen sich hierbei ergebender charakteristischer Verlauf der Mittelwerte an Festanteilen ist beispielsweise für die vorgenannte beispielhafte Lipidformulierung: 0 °C = 83%, 10 °C = 79%, 20 °C = 63%, 25 °C = 45%, 30 °C = 13% 32.5 ° = 6%, 35 °C = 4%, 37.5 °C = 2%, 40 °C = 0%. Die Lipidmischung liegt daher schon bei Temperaturen von 35 °C mit 96% und bei 37,5 °C mit 98% praktisch vollständig in flüssigem Zustand vor, während sie bei üblichen externen Lagerungstemperaturen physikalisch keine Fluiditätseigenschaften zeigt.

Um die Resorption insbesondere von Stoffen mit Peptid- oder Proteinstruktur zu verbessern, werden, in einer weiteren Ausbildung der Erfindung, als weitere Schutzstoffe Proteaseinhibitoren, insbesondere Aprotinin, in die Lipidmasse eingegeben.

Um in der Lipidmasse suspendierte oder emulgierte Wirkstoffe zusätzlich zu stabilisieren wird, in einer weiteren Ausbildung der Erfindung, der Lipidmischung hochdisperses Siliciumdioxid in hydrophiler oder hydrophobierter Form in Konzentrationen von 1-5% zugefügt.

Um das technische Herstellungsverfahren zu vereinfachen, werden, in einer weiteren Ausbildung der Erfindung, die erwärmten und flüssigen wirkstoffhaltigen Lipidmassen direkt in vorgefertigte Kapseln aus Hart- oder Weichgelatine gegossen und dort zur Erstarrung gebracht.

Um die Therapie mit bisher primär nur parenteral gegebenen Arzneistoffen zu verbessern, werden in einer weiteren Ausbildung der Erfindung, insbesondere Arzneistoffe mit Peptid- oder Proteinstruktur, wie Insulin, Calcitonin, Atriales Natriuretisches Peptid (ANP), Interferon, Somatostatin und Encephaline eingesetzt.

Um die Therapie bisher primär parenteral gegebener oder oral nicht zufriedenstellend resorbierter Arzneistoffe zu verbessern, werden in einer weiteren Ausbildung der Erfindung, Antibiotika, insbesondere Cephalosporine, eingesetzt.

Um die Therapie bei Arzneistoffen, durch eine Reduktion der erforderlichen Dosis, somit Verringerung des Nebenwirkungspotentials zu verbessern, werden in einer weiteren Ausbildung der Erfindung, cardiovasculär wirksame Stoffe wie Betablocker, Calciumantagonisten und Diuretika, sowie Steroidhormone wie Estradiol, Progesteron, Testosteron und Cortisol eingesetzt.

Die mit der Erfindung erzielten Vorteile ergeben sich insbesondere dadurch, daß die Lipidmischung ein thermisch reversibles Fest-Flüssig System mit natürlichen permeationsfördernden Eigenschaften darstellt,

EP 0 403 748 B1

wobei die spezifische Zusammensetzung der Lipide ein gutes Aufahmevermögen für sowohl hydrophile als auch lipophile Wirkstoffe besitzt. Hierdurch sind keine zusätzlichen Penetrationsförderer, sogenannte chemische Enhancer, erforderlich. Dies schaltet auch die Gefahr von Unverträglichkeiten und lokalen Schädigungen der Mucosa, wie sie bei Enhancern besteht, aus.

Die günstigen Hydrophilie-Lipohilie-Verhältnisse vermeiden auch die Anwendung zusätzlicher ionogener oder nichtionogener Emulgatoren, Tenside oder Lösungsvermittler wie Polyethylenglykol-Verbindungen (PEG). Eine physiologische Emulgierung der im Gastrointestinum freigesetzten Lipide erfolgt durch die endogenen Gallensäuren.

Die eingesetzten Lipidformulierungen besitzen ausgezeichnete Verträglichkeit und die Ausgangstoffe entstammen natürlichen Herkunftsquellen wie Kokos- und Palmkernfetten. Die Lipidgemische folgen auch der USP XXI/National Formulary NF XVI 4. Suppl. der Monographie für 'Hard Fat'.

Bei Untersuchung einer in der vorbeschriebenen Art zusammengesetzten Lipidformulierung zur akuten oralen Toxizität (LD 50, nach OECD Nr. 401) an der Ratte lag die akute mittlere orale LD50 über 2000 mg/Kg Körpergewicht der Ratte (Limittest). Bei Untersuchung der Kontaktsensibilisierung im Versuchsmodell nach Magnusson & Kligman (nach OECD Nr. 406) ergab der Sensitisation Test für die gleiche Lipidmasse = 0% (0/20), und wurde als nichtreizend und nichtsensibilisierend auf der Meerschweinchenhaut klassifiziert. Eine akute dermale Toxizität an Ratten (nach OECD Nr.402) ergab eine mittlere LD 50 dermal über 2000 mg Kg/KGW (Limittest).

Da die Lipidformulierungen ausschließlich gesättigte Fettsäuren, enthalten, besitzen sie bei der Lagerung auch eine gute Stabilität gegenüber Luftsauerstoff und unterliegen hierdurch auch nicht der Ranziditätsgefahr. Eine weitere Schutzmaßnahme gegen Oxidation stellt die feste Umhüllung dar.

Da die wirkstoffhaltigen Lipidmischungen, wie mittels vorgenannter Untersuchung durch Thermoanalyse, Steigschmelzpunkt und Solid Fat Index bestätigt bei Körpertemperatur im Gastrointestinum im flüssigen Aggregatzustand vorliegen, spreiten sie sich bei dem rasch erfolgenden Austritt aus der Trägerumhüllung unmittelbar auf der Mucosa. Hierdurch entstehen auch gute Konzentrationsgradienten, da die Lipide nur partiell wasserlöslich sind und kaum verdünnt werden. Dies begünstigt auch eine rasche Resorption und der Expositionszeitraum für Inaktivierungen durch gastrointestinale Lipasen und Proteasen wird verkürzt.

Der Festzustand der Lipidmischung während der Lagerung verbessert andererseits die Stabilität der Wirkstoffe und pharmazeutischtechnische Verarbeitungsformen wie Suspensionen oder Emulsionen werden durch die Erstarrung mechanisch stabilisiert.

Sowohl die technische Verarbeitung der Lipidmassen als auch die Einbringung der Lipidmassen in die festen Umhüllungen ist mit üblichen pharmazeutisch-technischen Methoden ausführbar und ermöglicht eine ökonomische Massenfertigung.

Die in der oralen Lipidarzneiform eingesetzten Lipidmischungen ermöglichen auch eine verbesserte Effektivität von Wirkstoffen mit Peptid- und Proteinstruktur, die bisher nur parenteral, in Form von Injektionen appliziert werden können. Hier bietet sich die therapeutische Einsatzmöglichkeit, entsprechende Wirkstoffe den Patienten in oraler Form statt in Injektionsform zu verabreichen. Daß die Erzeugung biologischer Effekte auch über die orale Applikationsroute mittels der Lipidformulierug z.B. für das blutzuckersenkende Proteohormon Insulin, grundsätzlich möglich ist, sei an nachfolgenden Beispielen demonstriert, wobei diese Beispiele nur einer weiteren Erläuterung der Erfindung dienen ohne daß diese hierauf beschränkt ist.

Beispiel 1: Gesunden, nichtkonditionierten weißen Mäusen wurde Insulin peroral verabreicht. Hierzu wurden wässrige Insulin-Lösungen mit Insulinkonzentrationen von 100 IU/ml in einer Lipidmischung vorgenannt zusammengesetzter Art, die mit Zusatz von 3% hochdispersem Siliciumdioxid versetzt war, bei 38°C dispergiert, für mehrere Minuten mit Ultraschall emulgiert und hiernach erstarrt. Mikroskopische Überprüfungen im Ausstrichpräparat der Lipidmasse sowie im doppelt gefärbten wässrigen Suspensionspräparat zeigten sphärisch-vesiculäre W/O-Komplexe bzw. im wässrigen Präparat W/O/W-Komplexe.

Nach erneuter Erwärmung auf 38°C wurden an Gruppen von jeweils 3 nüchtern gehaltenen Mäusen mit peroralem Plastikkatheter 0,4 ml der Lipidgemische mit unterschiedlichen Insulinkonzentrationen, zwischen 9,5 IU und 0,7 IU Insulin/kg Körpergewicht, in den Magen instilliert. Die einzelnen Insulin-Dosierungen wurden über Verdünnung der Insulin-Ausgangslösungen, durch entsprechende Zugabe an weiterer Lipidlösung, erstellt. Für jede Wirkstoffgruppe erfolgte eine zeitgleiche separate und gleichgroße Kontrollgruppe, der das gleiche Volumen insulinfreier Lipidmischung appliziert wurde. Blutentnahmen erfolgten vor sowie 1, 2 und 4 Stunden nach Applikation aus den Schwanzvenen. Die Blutglukosekonzentrationen wurden reflektionsdensitometrisch aus dem Vollblut bestimmt:

4

| Insulindosis (IU/kg KGW) | Zeit (h post appl.) | Kontrolle (nur Lipid) | Wirkgruppe (Lipid + Insulin) |
|---|---|---|---|
| | | Blutglukose (mg/dl) (Mittelwert±Standardabweichung) | |
| 9,5 | 0 | 165± 8 | 145± 6 |
| | 1 | 168±15 | 26±13 |
| | 2 | 176± 2 | 21± 2 |
| | 4 | 136 + 21 | 24 + 7 |
| 1.9 | 0 | 80± 2 | 82±10 |
| | 1 | 103±12 | 45± 2 |
| | 2 | 97±10 | 67±15 |
| | 4 | 77 + 5 | 62 + 14 |
| 0,95 | 0 | 136±14 | 121±20 |
| | 1 | 147±19 | 82± 9 |
| | 2 | 137±10 | 99±21 |
| | 4 | 121 + 22 | 122 + 9 |
| 0,7 | 0 | 116±10 | 123± 9 |
| | 1 | 109± 9 | 104± 9 |
| | 2 | 108± 3 | 84±29 |
| | 4 | 107±17 | 107±40 |

Die perorale Anwendung des Insulins führte somit sowohl gegenüber den zeitgleichen Werten der Kontrollgruppe als auch den Ausgangswerten der Wirkgruppe zu einer signifikanten, reproduzierbaren und dosisabhängigen Senkung der Blutglukose. Mit oral verabreichten, unveränderten, wässrigen Insulinlösungen ließ sich kein entsprechender blutzuckersenkender Effekt nachweisen.

Beispiel 2: In einer humanpharmakologischen Untersuchung erhielten 9 gesunde, männliche Probanden entweder subtherapeutische Insulindosen (Humaninsulin regular), durchschnittlich 0,2 oder 0,4 IU/kg Körpergewicht, oder die gleiche Lipidformulierung ohne Insulin (Placebo) peroral. Hierzu befand sich die erstarrte Insulin-Lipidemulsion, die in gleicher Weise wie in Beispiel 1 hergestellt wurde, in Hartgelatine Kapseln. Jeweils 3 Probanden erhielten 16 IU Insulin (8 IU/Kapsel) oder 32 IU und 3 Probanden erhielten Kapseln mit Placebo. Alle Probanden waren über Nacht nüchtern geblieben und erhielten, eine Stunde nach Applikation der Kapseln, in stündlichem Abstand 20 g standardisierte Kohlenhydrate. Die Serum-Insulinkonzentrationen wurden mittels Radioimmunoassay bestimmt.

| Stunden nach Applikation | | | | | | | |
|---|---|---|---|---|---|---|---|
| | 0 | 0,5 | 1 | 2 | 3 | 4 | 6 |
| Insulinmittelwerte im Serum (μU/ml) | | | | | | | |
| Placebo | 4,8 | 5,3 | 5,3 | 6,9 | 7,1 | 7,1 | 4,7 |
| 16 IU | 5,8 | 5,8 | 6,1 | 8,6 | 9,6 | 8,5 | 6,8 |
| 32 IU | 6,1 | 6,3 | 7,0 | 8,8 | 8,1 | 13,6 | 9,8 |

Aus dem Gruppenvergleich der zeitlichen Verläufe der mittleren Insulin-Serumkonzentrationen ist ersichtlich, daß die Gruppen mit den Insulinformulierungen höhere Insulinkonzentrationen aufweisen als die Placebogruppe und auch die beiden Insulindosierungen untereinander Unterschiede zeigen. Hieraus lässt sich eine intestinale Aufnahme des Insulins bei diesen gesunden und normal gegenregulierenden Probanden folgern.

Aus den beiden vorgenannten Beispielen ist ersichtlich, daß mit dem Erfindungsgegenstand auch bei Molekülen mit Peptidcharakter, die in der Regel im Magen-Darmtrakt biologisch inaktiviert werden, mit einer oralen Anwendung biologische Effekte erzeugt werden können.

Zur Verbessrung der Resorption von Wirkstoffen, die nach oraler Anwendung nicht ausreichend bioverfügbar sind, wird die orale Lipidarzneiform aus der Kombination einer halbfesten, lipophilen Komponente mit einer festen, wasserlöslichen Komponente gebildet. Die halbfeste Komponente ist eine als Hartfett vorliegende homogene Lipidmischung mit thermisch reversiblem Fest/Flüssig Schmelzverhalten, die noch unter Körpertemperaturen zu mindestens 95 % flüssig ist, und aus einer Mischung von Monoacyl-, Diacyl- und Triacylglyceriden gesättigter Pflanzenfettsäuren mit Kettenlängen von 6 bis 18 C Atomen, vorzugsweise von 40 bis 60 % Monoacyl- und Diacylglyceriden mit 40 bis 60 % Triacylglyceriden besteht, in der die Wirkstoffe direkt gelöst, suspendiert oder emulgiert vorliegen. Die feste Komponente besteht aus einer, die Lipidmasse einschließenden, nicht diffusiblen, wasserlöslichen Umhüllung vorzugsweise aus Gelatine oder Stärke. Die Verwendung von Proteaseinhibitoren im Lipidgemisch kann Permeationskonditionen für Peptide und Proteine verbessern. Die Anwendung von hochdispersem Siliziumdioxid kann Suspensionsformulierungen stabilisieren. Physikalischchemische als auch biochemische Aspekte der oralen Lipidarzneiform ergeben Verbesserungen für die Resorption oral nicht ausreichend bioverfügbarer Arzneistoffe.

**Patentansprüche**

**Patentsprüche für folgende Vertragsstaaten : AT, BE, CH, LI, DE, FR, GB, IT, NL, SE**

1. Orale Lipidarzneiform, insbesondere zur verbesserten Resorption nach oraler Applikation nicht ausreichend bioverfügbarer Wirkstoffe, dadurch gekennzeichnet, daß die orale Lipidarzneiform aus der Kombination einer halbfesten, lipophilen Komponente mit einer festen, wasserlöslichen Komponente gebildet wird, wobei die halbfeste Komponente eine als Hartfett vorliegende homogene Lipidmischung mit thermisch reversiblem Fest/Flüssig Schmelzverhalten, die noch unterhalb der Körpertemperatur von 37 Grad Celsius zu mindestens 95 % in flüssigem Aggregatzustand ist und aus Monoacyl-, Diacyl-, und Triacylglyceriden gesättigter Pflanzenfettsäuren der Kettenlängen von 6 bis 18 C Atomen, vorzugsweise der Mischung von 40 bis 60 % Monoacyl- und Diacylglyceriden mit 40 bis 60 % Triacylglyceriden, besteht, in der die Wirkstoffe entweder direkt gelöst, suspendiert oder emulgiert vorliegen, wobei die feste Komponente der Arzneiform aus einer die gesamte Lipidmasse einschließenden, nicht diffusiblen, wasserlöslichen und mit der Lipidmasse chemisch nicht verbundenen Hülle vorzugsweise aus Hart-, Weichgelatine oder Stärke besteht, die auch eine Schutzschicht gegen den Magensaft enthalten kann.

2. Orale Lipidarzneiform nach dem vorhergehenden Anspruch, dadurch gekennzeichnet, daß die im Gesamtlipidgemisch eingesetzten prozentualen Anteile an Fettsäuren für Caprylsäure 10-18 %, für Caprinsäure 5-15 %, für Laurinsäure 45-55 %, für Myristinsäure 9-15%, für Palmitinsäure 3-10% und für Stearinsäure 3-10 % betragen und diese Fettsäuren als Bestandteile von sowohl Monoacyl-, Diacyl- und Triacylglyceriden vorliegen.

3. Orale Lipidarzneiform, nach vorhergehenden Ansprüchen, dadurch gekennzeichnet, daß zur Verbesserung der Resorption von Stoffen mit Peptid- oder Proteinstrukturen als weitere Schutzstoffe Proteaseinhibitoren, insbesondere Aprotinin, in die Lipidmasse gegeben werden.

4. Orale Lipidarzneiform, nach vorhergehenden Ansprüchen, dadurch gekennzeichnet, daß zur Stabilisierung von Suspensionen und Emulsionen der Lipidmischung hochdisperses Siliziumoxid in hydrophiler oder hydrophobierter Form in Konzentrationen von 1-5% zugefügt wird.

5. Orale Lipidarzneiform, nach vorhergehenden Ansprüchen, dadurch gekennzeichnet, daß zur Vereinfachung des technischen Herstellungsverfahrens die erwärmten und flüssigen wirkstoffhaltigen Lipidmischungen direkt in vorgefertigte Kapseln aus Weich- oder Hargelatine gegossen und in diesen zur Erstarrung gebracht werden.

6. Orale Lipidarzneiform, nach vorhergehenden Ansprüchen, dadurch gekennzeichnet, daß als Wirkstoffe insbesondere Stoffe mit Peptid-oder Proteinstruktur wie Insulin, Calcitonin, Atriales Natriuretisches Peptid (ANP), Interferon, Somatostatin und Encephaline eingesetzt werden.

7. Orale Lipidarzneiform, nach vorhergehenden Ansprüchen, dadurch gekennzeichnet, daß orale Antibiotika, insbesondere Cephalosporine, eingesetzt werden.

8. Orale Lipidarzneiform, nach vorhergehenden Ansprüchen, dadurch gekennzeichnet, daß cardiovasculär wirksame Arzneistoffe wie Betablocker, Calciumantagonisten und Diuretika und Steroidhormone wie Estradiol, Progesteron, Testosteron und Cortisol eingesetzt werden.

9. Orale Lipidarzneiform nach vorhergehenden Ansprüchen, dadurch gekennzeichnet, daß die Lipidmischung bei der physiologischen menschlichen Körpertemperatur um 37 Grad Celsius zu mindestens 95

6

% im flüssigen Aggregatzustand vorliegt.

**Patentsprüche für folgenden Vertragsstaat : ES**

1. Verfahren zur Herstellung einer oralen Lipidarzneiform, insbesondere zur verbesserten Resorption nach oraler Applikation nicht ausreichend bioverfügbarer Wirkstoffe, dadurch gekennzeichnet, daß die orale Lipidarzneiform aus der Kombination einer halbfesten, lipophilen Komponente mit einer festen, wasserlöslichen Komponente gebildet wird, wobei als halbfeste Komponente eine als Hartfett vorliegende homogene Lipidmischung mit thermisch reversiblem Fest/Flüssig Schmelzverhalten, die noch unterhalb der Körpertemperatur von 37 Grad Celsius zu mindestens 95 % in flüssigem Aggregatzustand ist und aus Monoacyl-, Diacyl-, und Triacylglyceriden gesättigter Pflanzenfettsäuren der Kettenlängen von 6 bis 18 C Atomen, vorzugsweise der Mischung von 40 bis 60 % Monoacyl- und, Diacylglyceriden mit 40 bis 60 % Triacylglyceriden, verwendet wird, in der die Wirkstoffe entweder direkt gelöst, suspendiert oder emulgiert werden, und wobei als feste Komponente der Arzneiform eine die gesamte Lipidmasse einschließende , nicht diffusible , wasserlösliche und mit der Lipidmasse chemisch nicht verbundene Hülle, vorzugsweise aus Hart-, Weichgelatine oder Stärke verwendet wird, die auch eine Schutzschicht gegen den Magensaft enthalten kann.

2. Verfahren nach dem vorhergehenden Anspruch, dadurch gekennzeichnet, daß die im Gesamtlipidgemisch eingesetzten prozentualen Anteile an Fettsäuren für Caprylsäure 10-18 %, für Caprinsäure 5-15 %, für Laurinsäure 45-55 %, für Myristinsäure 9-15%, für Palmitinsäure 3-10% und für Stearinsäure 3-10 % betragen und diese Fettsäuren als Bestandteile von sowohl Monoacyl-, Diacyl- und Triacylglyceriden vorliegen.

3. Verfahren nach einem der vorhergehenden Ansprüche , dadurch gekennzeichnet, daß zur Verbesserung der Resorption von Stoffen mit Peptid- oder Proteinstrukturen als weitere Schutzstoffe Proteaseinhibitoren, insbesondere Aprotinin, in die Lipidmasse gegeben werden.

4. Verfahren nach einem der vorhergehenden Ansprüche , dadurch gekennzeichnet, daß zur Stabilisierung von Suspensionen und Emulsionen der Lipidmischung hochdisperses Siliziumoxid in hydrophiler oder hydrophobierter Form in Konzentrationen von 1-5% zugefügt wird.

5. Verfahren nach einem der vorhergehenden Ansprüche , dadurch gekennzeichnet, daß zur Vereinfachung des technischen Herstellungsverfahrens die erwärmten und flüssigen wirkstoffhaltigen Lipidmischungen direkt in vorgefertigte Kapseln aus Weich- oder Hargelatine gegossen und in diesen zur Erstarrung gebracht werden.

6. Verfahren nach einem der vorhergehenden Ansprüche , dadurch gekennzeichnet, daß als Wirkstoffe insbesondere Stoffe mit Peptid- oder Proteinstruktur wie Insulin, Calcitonin, Atriales Natriuretisches Peptid (ANP), Interferon, Somatostatin und Encephaline eingesetzt werden.

7. Verfahren nach einem der vorhergehenden Ansprüche , dadurch gekennzeichnet, daß orale Antibiotika, insbesondere Cephalosporine, eingesetzt werden.

8. Verfahren nach einem der vorhergehenden Ansprüche , dadurch gekennzeichnet, daß cardiovasculär wirksame Arzneistoffe wie Betablocker, Calciumantagonisten und Diuretika und Steroidhormone wie Estradiol, Progesteron, Testosteron und Cortisol eingesetzt werden.

9. Verfahren nach einem der vorhergehenden Ansprüche , dadurch gekennzeichnet, daß die Lipidmischung bei der physiologischen menschlichen Körpertemperatur um 37 Grad Celsius zu mindestens 95 % im flüssigen Aggregatzustand vorliegt.

**Claims**
**Claims for the following Contracting States : AT, BE, CH, LI, DE, FR, GB, IT, NL, SE**

1. Oral lipid pharmaceutical form, in particular for improved absorption after oral administration of inadequately bioavailable active ingredients, characterized in that the oral lipid pharmaceutical form is formed from the combination of a semi-solid, lipophilic component with a solid, water-soluble component, the semi-solid component being a homogeneous lipid mixture present as a hard fat and having thermally reversible solid/liquid melt behaviour, which is still in the liquid aggregate state to at least 95% below the body temperature of 37 degrees Celsius and consists of monoacyl-, diacyl- and triacylglycerides of saturated vegetable fatty acids of chain lengths from 6 to 18 carbon atoms, preferably the mixture of 40 to 60% of monoacyl- and diacylglycerides with 40 to 60% of triacylglycerides, in which the active ingredients are present either dissolved directly, suspended or emulsified, the solid component of the pharmaceutical form consisting of a non-diffusible, water-soluble shell enclosing the entire lipid mass and chemically not associated with the lipid mass, preferably of hard gelatin, soft gelatin or starch, which can also contain a protective layer against the gastric juice.

2. Oral lipid pharmaceutical form according to the preceding claim, characterized in that the percentage proportions of fatty acids employed in the total lipid mixture are 10-18% for caprylic acid, 5-15% for capric acid, 45-55% for lauric acid, 9-15% for myristic acid, 3-10% for palmitic acid and 3-10% for stearic acid and these fatty acids are present as constituents of monoacyl-as well as diacyl- and triacylglycerides.

3. Oral lipid pharmaceutical form, according to the preceding claims, characterized in that, to improve the absorption of substances having peptide or protein structures, protease inhibitors, in particular aprotinin, are added to the lipid mass as further protective substances.

4. Oral lipid pharmaceutical form, according to the preceding claims, characterized in that, to stabilize suspensions and emulsions of the lipid mixture, highly disperse silica in hydrophilic or hydrophobized form is added in concentrations of 1-5%.

5. Oral lipid pharmaceutical form, according to the preceding claims, characterized in that, to simplify the industrial production process, the warmed and liquid active ingredient-containing lipid mixtures are poured directly into previously prepared soft gelatin or hard gelatin capsules and solidified in these.

6. Oral lipid pharmaceutical form, according to the preceding claims, characterized in that, as active ingredients, substances having a peptide or protein structure such as insulin, calcitonin, atrial natriuretic peptide (ANP), interferon, somatostatin and encephalins are in particular employed.

7. Oral lipid pharmaceutical form, according to the preceding claims, characterized in that oral antibiotics, in particular cephalosporins, are employed.

8. Oral lipid pharmaceutical form, according to the preceding claims, characterized in that pharmaceutical substances having cardiovascular activity, such as beta-blockers, calcium antagonists and diuretics and steroid hormones such as oestradiol, progesterone, testosterone and cortisol are employed.

9. Oral lipid pharmaceutical form, according to the preceding claims, characterized in that the lipid mixture is present to at least 95% in the liquid aggregate state at the physiological human body temperature of around 37 degrees Celsius.

**Claims for the following Contracting State : ES**

1. Process for the preparation of an oral lipid pharmaceutical form, in particular for improved absorption after oral administration of inadequately bioavailable active ingredients, characterized in that the oral lipid pharmaceutical form is formed from the combination of a semi-solid, lipophilic component with a solid, water-soluble component, the semi-solid component used being a homogeneous lipid mixture present as a hard fat and having thermally reversible solid/liquid melt behaviour, which is still in the liquid aggregate state to at least 95% below the body temperature of 37 degrees Celsius and consisting of monoacyl-, diacyl- and triacylglycerides of saturated vegetable fatty acids of chain lengths from 6 to 18 carbon atoms, preferably the mixture of 40 to 60% of monoacyl- and diacylglycerides with 40 to 60% of triacylglycerides, in which the active ingredients are either dissolved directly, suspended or emulsified, and the solid component of the pharmaceutical form used being a non-diffusible, water-soluble shell enclosing the entire lipid mass and chemically not associated with the lipid mass, preferably of hard gelatin, soft gelatin or starch, which can also contain a protective layer against the gastric juice.

2. Process according to the preceding claim, characterized in that the percentage proportions of fatty acids employed in the total lipid mixture are 10-18% for caprylic acid, 5-15% for capric acid, 45-55% for lauric acid, 9-15% for myristic acid, 3-10% for palmitic acid and 3-10% for stearic acid and these fatty acids are present as constituents of monoacyl- as well as diacyl- and triacylglycerides.

3. Process according to one of the preceding claims, characterized in that, to improve the absorption of substances having peptide or protein structures, protease inhibitors, in particular aprotinin, are added to the lipid mass as further protective substances.

4. Process according to one of the preceding claims, characterized in that, to stabilize suspensions and emulsions of the lipid mixture, highly disperse silica in hydrophilic or hydrophobized form is added in concentrations of 1-5%.

5. Process according to one of the preceding claims, characterized in that, to simplify the industrial production process, the warmed and liquid active ingredient-containing lipid mixtures are poured directly into previously prepared soft gelatin or hard gelatin capsules and solidified in these.

6. Process according to one of the preceding claims, characterized in that, as active ingredients, substances having a peptide or protein structure such as insulin, calcitonin, atrial natriuretic peptide (ANP), interferon, somatostatin and encephalins are in particular employed.

7. Process according to one of the preceding claims, characterized in that oral antibiotics, in particular cephalosporins, are employed.

8. Process according to one of the preceding claims, characterized in that pharmaceutical substances having cardiovascular activity, such as beta-blockers, calcium antagonists and diuretics and steroid hormones such as oestradiol, progesterone, testosterone and cortisol are employed.

9. Process according to one of the preceding claims, characterized in that the lipid mixture is present to at least 95% in the liquid aggregate state at the physiological human body temperature of around 37 degrees Celsius.

**Revendications**
**Revendications pour les Etats contractants suivants : AT, BE, CH, LI, DE, FR, GB, IT, NL, SE**

1. Présentation médicamenteuse lipidique orale, en particulier pour améliorer la résorption après administration orale de substances actives insuffisamment biodisponibles, caractérisée en ce que la présentation médicamenteuse lipidique orale est formée de l'association d'un constituant semi-solide, lipophile et d'un constituant solide, soluble dans l'eau, le constituant semi-solide étant un mélange homogène se présentant comme une graisse dure, ayant un comportement à la fusion solide/liquide thermiquement réversible, qui est encore au-dessous de la température du corps de 37°C, à 95 % au moins à l'état d'agrégat liquide et se compose de monoacyl-, diacyl- et triacylglycérides d'acides gras végétaux saturés ayant une longueur de chaîne de 6 à 18 atomes de C, de préférence du mélange de 40 à 60 % de monoacyl- et de diacylglycérides avec 40 à 60 % de triacylglycérides, dans lequel les substances actives sont soit directement dissoutes, mises en suspension ou émulsionnées, le constituant solide de la présentation médicamenteuse étant constitué d'une enveloppe renfermant la totalité de la masse lipidique, non diffusible, soluble dans l'eau et non liée chimiquement à la masse lipidique, de préférence en gélatine dure, en gélatine molle ou en amidon, qui peut aussi contenir une couche protectrice contre le suc gastrique.

2. Présentation médicamenteuse lipidique orale selon la revendication précédente, caractérisée en ce que les proportions en pourcentage d'acides gras utilisées dans le mélange lipidique total s'élèvent pour l'acide caprylique à 10 à 18 %, pour l'acide caprique à 5 à 15 %, pour l'acide laurique à 45 à 55 %, pour l'acide myristique à 9 à 15 %, pour l'acide palmitique à 3 à 10 % et pour l'acide stéarique à 3 à 10 %, et en ce que ces acides gras sont présents comme constituants

3. Présentation médicamenteuse lipidique orale selon les revendications précédentes, caractérisée en ce que pour améliorer la résorption de substances ayant une structure peptidique ou protéinique, on ajoute dans la masse lipidique, comme substances protectrices supplémentaires, des inhibiteurs de protéases, en particulier l'aprotinine.

4. Présentation médicamenteuse lipidique orale selon les revendications précédentes, caractérisée en ce que pour la stabilisation de suspensions et d'émulsions, on ajoute au mélange lipidique de l'oxyde de silicium hautement dispersé, sous une forme hydrophile ou hydrophobisée, à une concentration de 1 à 5 %.

5. Présentation médicamenteuse lipidique orale selon les revendications précédentes, caractérisée en ce que pour simplifier le procédé de préparation technique, on introduit directement les mélanges lipidiques contenant des substances actives, chauffés et liquides, dans des capsules fabriquées à l'avance en gélatine molle ou dure et en ce qu'on les fait solidifier dans celles-ci.

6. Présentation médicamenteuse lipidique orale selon les revendications précédentes, caractérisée en ce qu'on utilise comme substances actives en particulier des substances ayant une structure peptidique ou protéinique telles que l'insuline, la calcitonine, le peptide natriurétique auriculaire (ANP), l'interféron, la somatostatine et les encéphalines.

7. Présentation médicamenteuse lipidique orale selon les revendications précédentes, caractérisée en ce qu'on utilise des antibiotiques oraux, en particulier des céphalosporines.

8. Présentation médicamenteuse lipidique orale selon les revendications précédentes, caractérisée en ce qu'on utilise des médicaments ayant une action cardiovasculaire tels que des béta-bloquants, des antagonistes du calcium et des diurétiques, et des cardiovasculaire tels que des béta-bloquants, des antagonistes du calcium et des diurétiques, et des hormones stéroidiennes, telles que l'oestradiol, la progestérone, la testostérone et le cortisol.

9. Présentation médicamenteuse lipidique orale selon les revendications précédentes, caractérisée en ce que le mélange lipidique est présent, à 95 % au moins à l'état d'agrégat liquide, à la température physiologique du corps humain de 37 °C.

**Revendications pour l'Etat contractant suivant : ES**

1. Procédé de préparation d'une présentation médicamenteuse lipidique orale, en particulier pour améliorer la résorption après administration orale de substances actives insuffisamment biodisponibles, caractérisé en ce que la présentation médicamenteuse lipidique orale est formée de l'association d'un constituant semi-solide, lipophile et d'un constituant solide, soluble dans l'eau, en utilisant comme constituant semi-solide un mélange lipidique homogène se présentant sous la forme d'une graisse dure ayant un comportement à la fusion solide/liquide réversible, qui est encore à 95 % au moins à l'état d'agrégat liquide au-dessous de la température corporelle de 37 °C et se compose de monoacyl- diacyl- et triacylglycérides d'acides gras végétaux saturés ayant une longueur de chaîne de 6 à 18 atomes de C, de préférence du mélange de 40 à 60 % de monoacyl- et de diacylglycérides contenant 40 à 60 % de triacyl-glycérides, dans lequel les substances actives sont soit directement dissoutes, mises en suspension, soit émulsionnées, et en utilisant comme constituant solide de la présentation médicamenteuse une enveloppe renfermant la totalité de la masse lipidique, non diffusible, soluble dans l'eau et non liée chimiquement à la masse lipidique, de préférence en gélatine dure, en gélatine molle ou en amidon, qui peut contenir également une couche protectrice contre le suc gastrique.

2. Procédé selon la revendication précédente, caractérisé en ce que les proportions en pourcentage des acides gras utilisés dans le mélange lipidique total sont de 10 à 18 % pour l'acide caprylique, de 5 à 15 % pour l'acide caprique, de 45 à 55 % pour l'acide laurique, de 9 à 15 % pour l'acide myristique, de 3 à 10 % pour l'acide palmitique et de 3 à 10 % pour l'acide stéarique, et en ce que ces acides gras sont présents en tant que constituants tant de monoacyl- que de diacyl- et de triacylglycérides.

3. Procédé selon l'une des revendications précédentes, caractérisé en ce que pour améliorer la résorption de substances ayant des structures peptidiques ou protéiniques, on ajoute à la masse lipidique, comme autres substances protectrices, des inhibiteurs de protéases, en particulier de l'aprotinine.

10

4. Procédé selon l'une des revendications précédentes, caractérisé en ce que pour stabiliser les suspensions et les émulsions, on ajoute au mélange lipidique de l'oxyde de silicium hautement dispersé sous forme hydrophile ou hydrophobisée à une concentration de 1 à 5 %.

5. Procédé selon l'une des revendications précédentes, caractérisé en ce que pour simplifier le procédé technique de fabrication, on verse les mélanges lipidiques contenant les substances actives, chauffés et liquides directement dans des capsules préfabriquées en gélatine molle ou dure et on les fait solidifier dans celles-ci.

6. Procédé selon l'une des revendications précédentes, caractérisé en ce qu'on utilise en particulier comme substances actives des substances ayant une structure peptidique ou protéinique telles que l'insuline, la calcitonine, le peptide natriurétique auriculaire (ANP), l'interféron, la somatostatine et des encéphalines.

7. Procédé selon l'une des revendications précédentes, caractérisé en ce qu'on utilise des antibiotiques oraux, en particulier des céphalosporines.

8. Procédé selon l'une des revendications précédentes, caractérisé en ce qu'on utilise des médicaments ayant une action cardiovasculaire tels que des béta-bloquants, des antagonistes du calcium et des diurétiques et des hormones stéroidiennes, telles que l'oestradiol, la progestérone, la testostérone, et le cortisol.

9. Procédé selon l'une des revendications précédentes, caractérisé en ce que le mélange lipidique, à la température physiologique du corps humain de 37°C, est présent à 95 % au moins à l'état d'agrégat liquide.